Europäisches Patentamt

European Patent Office

Office européen des brevets

(11) Publication number: **0 462 651 A1**

# EUROPEAN PATENT APPLICATION

(21) Application number: 91201447.9

(51) Int. Cl.⁵: **C12N 15/58**, C12N 9/72, C12N 5/10, A61K 37/54

(22) Date of filing: **11.06.91**

(30) Priority: **15.06.90 US 538458**

(43) Date of publication of application:
**27.12.91 Bulletin 91/52**

(84) Designated Contracting States:
**AT BE CH DE DK ES FR GB GR IT LI LU NL SE**

(71) Applicant: **Leuven Research & Development V.Z.W.**
**Benedenstraat 59A Groot Begijnhof**
**B-3000 Leuven(BE)**

(72) Inventor: **Collen, Désiré J.**
**Schoonzichtlaan 2**
**B-Winksele(BE)**

Inventor: **Lijnen, Roger H.**
**Acacialaan 50A**
**B-Herent(BE)**
Inventor: **Nelles, Lucien G.R.**
**Dellestraat 20**
**B-Herent(BE)**
Inventor: **Stassen, Jean-Marie E.I.**
**Jozef Ravoetstraat 5**
**B-Wilsele(BE)**

(74) Representative: **Bruin, Cornelis Willem et al**
**OCTROOIBUREAU ARNOLD & SIEDSMA**
**Sweelinckplein 1**
**NL-2517 GK The Hague(NL)**

(54) **Chimeric plasminogen activators.**

(57) Chimeric plasminogen activators comprising at least the enzymatic region of urokinase-type plasminogen activator in combination with polypeptide material from another source, will sometimes show improved thrombolytic potency in vivo. The other polypeptide material is preferably a fragment, without substantial fibrin-affinity from the non-enzymatic portion of tissue-type plasminogen activator. The most preferred product is a chimeric molecule comprising aminoacids 1 to 3 and 87 to 274 of tissue-type plasminogen activator fused to aminoacids 138 to 411 of urokinase-type plasminogen activator. These products may occur in single-chain or two-chain form and may be used in pharmaceutical compositions for the treatment of thromboembolic diseases.

FIG.3

This invention relates to plasminogen activators, their production and utilisation. More in particular, it relates to new plasminogen activators which are chimeras of plasminogen activators of the urokinase-type with polypeptide structures which endow the molecule with improved in vivo thrombolytic potency, as well as to methods for the preparation of these new plasminogen activators, pharmaceutical compositions containing the same, and methods of using such plasminogen activators in the treatment of patients with thromboembolic diseases.

It is known that certain enzymes, called plasminogen activators, after intravenous infusion in man or in animals, can exert a thrombolytic action in vivo. This action is based on activation of plasminogen present in blood, which initiates a chain of reactions resulting in the lysis of a fibrin-containing blood clot in the blood vessels. The mechanisms of action are complex and can be different depending on the type of plasminogen activator which is used. Human plasminogen activators can be differentiated into two types, i.e., plasminogen activators of the tissue-type (t-PA) and plasminogen activators of the urokinase-type (u-PA). The plasminogen activators of the urokinase-type can be subdivided into a two-chain form (tcu-PA) and a one-chain form (scu-PA) (Collen, J.Cell. Biochem. 33, 77-86, 1987).

For a good understanding of the following, reference is made to the accompanying drawings, wherein:
fig. 1 shows the primary structure of human t-PA,
fig. 2 shows the primary structure of human scu-PA,
fig. 3 is a diagrammatic representation of the chimeric plasminogen activators used in the Examples of this specification.

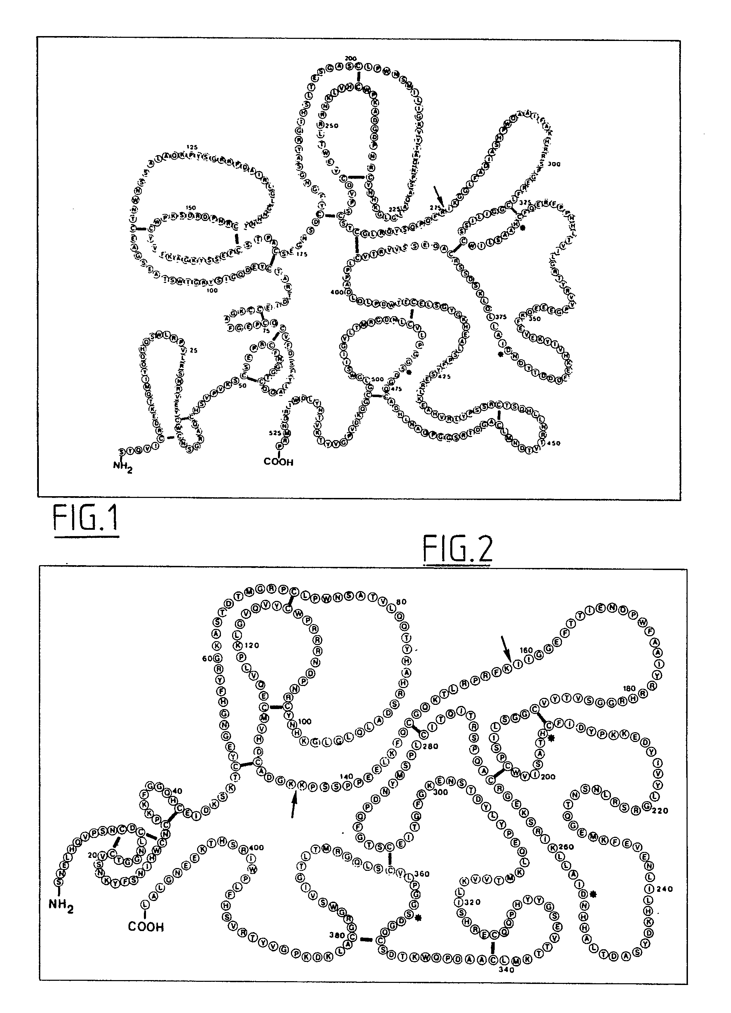

According to fig. 1, human t-PA is a serine protease composed of one polypeptide chain containing 527 amino acids. These amino acids have been numbered by the system of Pennica et al, Nature 301, 214-221, 1983. Limited plasmic hydrolysis of the $Arg^{275}$-$Ile^{276}$ peptide bond (indicated by an arrow) will convert the molecule into a two-chain activator linked by one disulfide bond. The catalytic site of t-PA is composed of residues His, Asp and Ser in positions 322, 371 and 478, respectively, which have been indicated by an asterisk in fig. 1.

The t-PA molecule has four specific domains, viz.:
1) a 43-residue-long amino-terminal region which is homologous with the finger-domains responsible for fibrin affinity of fibronectin;
2) residues 44-91 which are homologous with human epidermal growth factor;
3) two regions of 82 amino acids each (residues 92-173 and 180-261) which share a high degree of homology with the five kringles of plasminogen; and
4) a serine protease part with the active site residues His, Asp and Ser. In this specification, these four domains will be indicated as (1) finger domain (F-domain); (2) epidermal growth factor domain (E-domain); (3) kringle domain K1 and kringle domain K2; and (4) the catalytic domain.

According to fig. 2, human scu-PA is a serine protease composed of a single polypeptide chain containing 411 amino acids with 24 cysteine residues. The amino acid residues have been numbered by the system of Holmes et al, Biotechnology, 3, 923-929, 1985. Hydrolysis of the $Lys^{158}$-$Ile^{159}$ peptide bond (indicated by an arrow) by means of plasmin will convert the molecule to urokinase, a two-chain molecule linked by one disulfide bridge.

The scu-PA molecule has three domains, viz.: a cysteine-rich amino-terminal region (residues 5-49) which is homologous with epidermal growth factor; a kringle region comprising amino acid residues 50-136, and a serine protease part with the active site residues His, Asp and Ser in positions 204, 255 and 356 resp., which constitutes the carboxy-terminal region of the molecule. In this specification, the three domains will be indicated as: epidermal growth factor domain (E-domain); kringle domain; and catalytic domain or protease domain, resp. The active site has been indicated by an asterisk in fig. 2.

The tissue-type plasminogen activator (t-PA) can cause activation of plasminogen but it is much more efficient in the presence of fibrin than in its absence. This means that activation mainly occurs in the vicinity of a blood clot, which is dissolved, while much less activation of plasminogen occurs in circulating blood. The fibrin-specificity of t-PA is mainly due to its affinity for fibrin and to a remarkably enhanced activation of plasminogen at the fibrin surface. Structures in t-PA responsible for its fibrin-affinity are localized in its $NH_2$-terminal region, probably in the finger-like domain and in the second kringle. t-PA does not react with antisera aginst tcu-PA or scu-PA.

The single chain urokinase-type plasminogen activator (scu-PA) activates plasminogen efficiently, but only in the presence of fibrin-containing blood clots. Likewise as with t-PA, less activation of the fibrinolytic system in circulating blood will occur, notwithstanding the fact that the mechanism of activation is different; u-PA only displays fibrin-specificity in its scu-PA form, in which the $Lys^{158}$-$Ile^{159}$ peptide bond is intact. The fibrin-specificity is not dependent on the presence of the $NH_2$-terminal 143 amino acids. Contrary thereto, tcu-PA (urokinase) induces activation of plasminogen in blood both in the presence and in the absence of fibrin. This means that blood clots can be dissolved, but also that activation of the fibrinolytic system in circulating blood occurs, which may lead to fibrinogen breakdown and a bleeding tendency (Collen, J. Cell. Biochem. 33, 77-86, 1987).

Among these three human plasminogen activators (t-PA, scu-PA and tcu-PA) , tcu-PA has been used in medical practice already for a long time, during which the described side effects have been a handicap. t-PA has been approved as an improved thrombolytic agent since 1987. Although many questions relating to the optimal use of these active compounds remain unanswered hitherto, it has been established that the fibrin-specificity of t-PA and scu-PA in man is not as pronounced as could be expected from animal models.

Both t-PA and u-PA have a very short plasma half-life as a result of rapid hepatic clearance, and consequently, their therapeutic use requires continuous intravenous infusion of relatively large amounts of material, which is time-consuming and expensive. Therefore, the quest for thrombolytic agents having a higher thrombolytic potency, a higher specific thrombolytic activity and/or a better fibrin-selectivity continues (Collen et al., Ann. Rev. Med., 39, 405-423, 1988).

One approach to the development of improved thrombolytic agents might consist in the production, by chemical cross-linking or recombinant DNA methods, of chimeric proteins which contain the structures responsible for the fibrin-specificity of both t-PA and scu-PA. This approach has been used already by several groups as reviewed by Haber et al (Science 243, 51-56, 1989), by Krause (Fibrinolysis 2, 133-142, 1988) and by Lijnen and Collen (Blut 57, 147-162, 1988). Chimeric plasminogen activators are molecules which contain structures harbouring the enzymatic activity of t-PA or u-PA, in combination with amino acid sequences derived from other proteins. The latter structures were selected in order to increase the fibrin-selectivity of the molecules and have comprised e.g. finger-like domains of t-PA, or kringle domains of t-PA, u-PA, prothrombin or plasmin, or fibrin-specific antibodies.

u-PA based chimeras have been produced by disulfide cross-linkage between the A-chain of plasmin and the B-chain of urokinase (Robbins and Tanaka, Biochemistry 25, 3603-3611, 1986 European Patent Application EP-A-0250071; EP-A-0290118; EP-A-0297882), or between the A-chain of t-PA and the B-chain of urokinase (European Patent Application EP-A-0155387). Other chimeric molecules combine parts of the NH$_2$-terminal sequence of t-PA or plasmin fused to the COOH-terminal protease region of u-PA by recombinant DNA technology; Nelles et al, J. Biol. Chem. 262, 10855-18862, 1987; de Vries et al Biochemistry 27, 2565-2572, 1988; Pierard et al, DNA 8, 321-328, 1989; Lee et al, J. Biol. Chem. 263, 2917-2924, 1988; Rajput et al, Thromb. Haemost. 58, 438, 1987; Mao et al, Fibrinolysis 2, Suppl. 1, 50, 1988; Heim et al, Thromb. Haemost. 62, 337, 1989; Australian Patent Application AU-A-3707148; European Patent Application EP-A-0231794, EP-A-0273774, EP-A-0231883; PCT Patent Applications WO 88/08451 and WO 89/10401).

On the other hand, t-PA based chimeras have been constructed by disulfide cross-linkage of the A-chain of plasmin to the B-chain of t-PA (Robbins and Boreisha, Biochemistry 26, 4661-4667, 1987; European Patent Applications EP-A-0292326, EP-A-0290118, EP-A-0297882), by joining the NH$_2$-terminal portion of u-PA to a truncated t-PA molecule (European Patent Application EP-A-0213794; Lee et al, Thromb. Haemost. 58, 313, 1987), by inserting urokinase or prothrombin kringles on either side of or between the two kringles in the t-PA molecule (Lee et al, J. Biol. Chem. 263, 2917-2924, 1988), or by fusing a plasminogen kringle to a truncated t-PA molecule (Langer-Safer et al, Thromb. Haemost. 62, 337, 1989).

Most of these recombinant enzymes were shown to be enzymatically active, with some, but by far not all, retaining partial fibrin-affinity of t-PA, and with some, but not all, having a significantly delayed clearance. However, it has become obvious that domain shuffling does not increase the fibrin-selectivity beyond that attained by the natural plasminogen activators. Furthermore, notwithstanding years of intense work in this area, no chimeric molecules with markedly improved thrombolytic potency in vivo have been found.

In our laboratory, evidence was obtained that attempts to endow the enzymatically active region of scu-PA with fibrin-affinity by its conjugation or fusion with domains of the t-PA molecule tend to reduce, or at best only maintain, the thrombolytic potency of the chimeras relative to that of scu-PA. Thus, chimeric molecules of t-PA and u-PA were produced by transient expression of cDNA fragments of t-PA (encoding amino acids 1 to 67, 1 to 212 or 1 tot 313, respectively) fused to cDNA fragments of scu-PA (encoding amino acids 136 to 411, 139 to 411 or 195 to 411, respectively). These recombinant products, following cleavage by plasmin, activated plasminogen at a rate very similar to that of u-PA, which indicates that fusion of t-PA structures to u-PA does not impair the catalytic activity of the enzyme (Pierard et al, J. Biol. Chem., 262, 11771-11778, 1987). Further investigation with one of these chimeric molecules (amino acids 1 to 67 of t-PA and 136 to 411 of scu-PA) however, revealed that it had not acquired the fibrin-affinity of t-PA nor an improved in vitro fibrin-selectivity as compared to scu-PA (Gheysen et al, J. Biol . Chem. 262, 11779-11784, 1987). More recent additional work with some of these chimeras has demonstrated that fusion of the finger and/or kringle domain of t-PA to scu-PA does not endow the molecule with fibrin-affinity (Pierard et al, DNA,

8, 321-328, 1989).

Further in our laboratory, some chimeric plasminogen activators were produced by stable expression of cDNA in mammalian cell systems. Two chimeric plasminogen activators (t-PA/u-PA-s and t-PA/u-PA-e), consisting of the $NH_2$-terminal part of t-PA (amino acids 1 through 263 and 1 through 274 respectively), and the catalytic domain of scu-PA (amino acids 144 through 411 and 138 through 411 respectively) retained the specific activity, catalytic efficiency and fibrinolytic potency in vitro of u-PA (either in their single-chain or in their two-chain forms), but only acquired partially the fibrin-affinity and the fibrin stimulation of t-PA (Nelles et al, J. Biol. Chem. 262, 10855-10862, 1987; Lijnen et al, J. Biol. Chem. 263, 19083-19091, 1988).

Three other chimeric plasminogen activators which can be regarded as domain deletion/substitution variants of the t-PA/u-PA-e chimer were produced by us.

In these variants, the finger-like (F) and epidermal growth factor-like (E) domains had been deleted and/or the kringle 1 ($K_1$) had been substituted by a second copy of kringle 2 ($K_2$). It appeared that deletion of the F and E domains in t-PA/u-PA-e markedly reduced the fibrin-affinity thereof, whereas substitution of $K_1$ by a second copy of $K_2$ in the same molecule enhanced the affinity for fibrin significantly. However, neither modification significantly altered the specific activity, catalytic efficiency or fibrinolytic potency in a clot lysis system when tested in vitro.

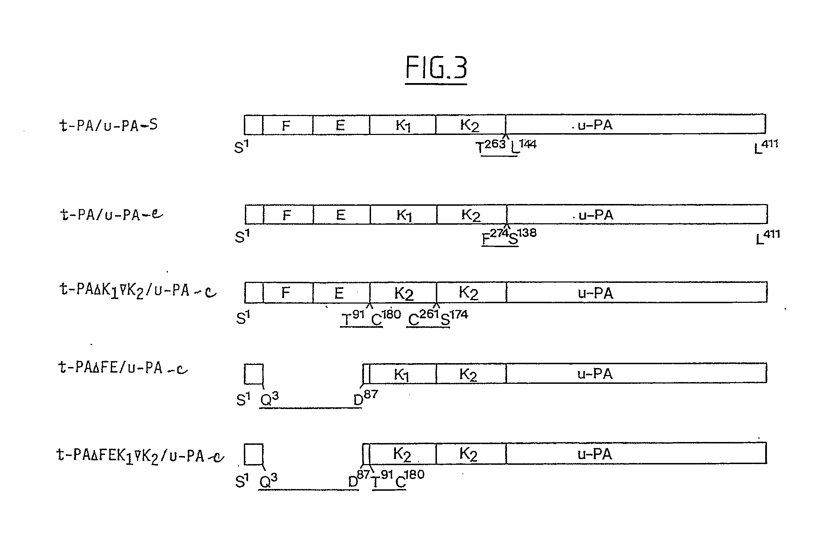

The structures of t-PA/u-PA-s, t-PA/u-PA-e and the three last-mentioned variants have been represented schematically by the bar diagram in fig. 3. Positions where the deletions and rearrangements occur have been indicated by the underlined amino acids.

Upon subsequent in vivo testing of the five last-mentioned chimeras, we have now quite surprisingly found that one of these chimeras, namely the variant of t-PA/u-PA-e with deletion of F and E domains (indicated as t-PA-$\Delta$FE/u-PA-e) has a markedly increased thrombolytic potency in vivo, compared with t-PA or scu-PA. This effect was found in a hamster pulmonary embolism model and confirmed in a rabbit jugular vein thrombosis model and a baboon femoral vein thrombosis model. Further, we found that the increased thrombolytic potency in vivo is mainly determined by a reduced clearance from the body in combination with a relative maintenance of the specific thrombolytic activity in vivo. The phenomenon of a markedly reduced clearance was also shown by two other variants of t-PA/u-PA-e (indicated as t-PA-$\Delta K_1 \nabla K_2$/u-PA-e and t-PA-$\Delta FEK_1 \nabla K_2$/u-PA-e) but they had a markedly reduced specific thrombolytic activity in addition thereto, thus resulting in a thrombolytic potency which was only comparable to or at best marginally higher than that of scu-PA. The invention is based on these findings.

A primary object of the invention is to provide new chimeric plasminogen activators that have an increased thrombolytic potency compared with t-PA or scu-PA. A related object is to provide methods for the production of such chimaric plasminogen activators.

A further object is to provide pharmaceutical compositions and/or clinical methods based on such chimeric plasminogen activators and being useful in the treatment of thromboembolic diseases.

The new chimeric plasminogen activators embraced by the invention include the above t-PA-$\Delta$FE/u-PA-e chimer as tested and any other chimer comprising the carboxyterminal region of single-chain urokinase-type plasminogen activator combined with a polypeptide structure capable of substantially reducing the clearance of the molecule while relatively maintaining its specific thrombolytic activity in vivo. More in general, the invention will include all chimeric plasminogen activators which comprise a polypeptide structure harboring the enzymatic activity of urokinase-type plasminogen activator, linked to a polypeptide structure capable of endowing the molecule with improved in vivo thrombolytic potency.

The new chimeric plasminogen activators can be produced by chemical methods or by recombinant DNA methods. Chemical production methods will include cross-linking, deletion and addition operations. Recombinant DNA methods will include: construction of cDNA clones encoding the desired chimera, construction of plasmids, expression of plasmids in suitable host cells and isolation and purification of the plasminogen activators produced by such cells. Suitable techniques for constructions of cDNA fragments and molecules are available to those skilled in the art. Although any suitable cell type may be used as host cells for expression of plasmids, Chinese hamster ovary cells (CHO cells) are preferred for this purpose. Isolation and purification of the plasminogen activators produced may be effected by known methods such as chromatography on Zinc Chelate Sepharose, immuno adsorption on insolubilized murine monoclonal antibodies, etc.

The chimeric plasminogen activators of the invention may be combined with a suitable excipient to provide a pharmaceutical composition useful in the treatment of thromboembolic diseases. The excipient may be any conventional and pharmaceutically acceptable medium for this purpose. Further, other plasminogen activators may be used concomitantly with the invented chimeric plasminogen activators, thus creating a possible synergistic effect. The resulting composition has preferably the form of an intravenous infusion fluid since this form has the best chances of providing efficient results, and administration may be effected either by continuous infusion or by bolus injection.

The invention is illustrated by the following examples whioh should not be read in a restricting sense.

Example I

Production of t-PA/u-PA-s and t-PA/u-PA-e

Two chimeric plasminogen activators, t-PA/u-PA-s and t-PA/u-PA-e were produced and purified in the way disclosed by Nelles et al, J. Biol. Chem. 262, 10855-10862, 1987 and by Lijnen et al, J. Biol. Chem. 263, 19083-19091, 1988. The various steps of the production process are repeated here for the sake of convenience.

Total RNA was isolated from Bowes human melanoma cells with the known guanidinium isothiocyanate extraction/lithiumchloride precipitation method (Cathalla et al., DNA 2, 329-335, 1983). Poly A+ mRNA was prepared by oligo-dT chromatography (Aviv and Leder, Proc. Natl. Acad. Sci. USA 69, 1408-1412, 1972). Oligo-dT-primed cDNA was prepared according to Gubler and Hoffman, (Gene 25, 263-269, 1983) and cloned in λgtll (Huynn et al, In "DNA cloning: a practical approach" Vol 1, pp 49-78, D.M. Glover, ed. IRC Press, Oxford, 1984).

Two partially overlapping deoxyoligonucleotides representing complementary strands of t-PA cDNA were chemically synthesized. These two strands 5'-GGGCACCTGCCAGCAGGCCCTGTACTTCTC-3' and 5'-GGGACTCGGCACACGAAATCTGAGAAGTAC-3' were annealed and filled in using the Klenow fragment of E. coli DNA polymerase I (final concentration 0.1 Units/ml, Boehringer Mannheim), and 200 $\mu$M of dGTP, dTTP and dATP (Pharmacia, Uppsala, Sweden) and 250 $\mu$Ci ($\alpha$-32P) dCTP, 5,000 Ci/mmole (New England Nuclear, Boston, MA) to produce a 51 nucleotide long probe with a specific activity of ~$10^8$ cpm/$\mu$g. This probe spans nucleotides 366 through 416 of the cDNA sequence (Pennica et al, Nature 301,214-221, 1983). Plaque hybridization on the Bowes melanoma cDNA library was performed with this probe.

cDNA from positive plaques (λt-PA4 and λt-PA8) were spliced together in pUC18 (Yanisch-Perron et al., Gene 33, 103-119, 1985) to produce a contiguous cDNA sequence encoding t-PA (pUL-t-PA). t-PA cDNA sequences were then subcloned into M13mp18 or M13mp19 (Yanisch-Perron et al., Gene 33, 103-119, 1985), and sequenced by the dideoxynucleotide chain termination method (Sanger et al., Proc. Natl. Acad. Sci. USA 74, 5463-5467, 1977).

The complete t-PA cDNA coding sequences, and additional 127 nucleotides of 5'-untranslated sequence and 391 nucleotides of 3'-untranslated sequence were isolated as a BamHI restriction endonuclease fragment from pULt-PA and cloned in the unique BamHI restriction site of the expression vector pSV328DHFR (Nelles et al. J. Biol. Chem. 262, 5682-5689, 1987) to yield pSVt-PA-DHFR.

I.2.Construction of u-PA cDNA clones

Total RNA was isolated from CALU-3 cells (ATCC, HTB-55) by the guanidinium isothiocyanate extraction/lithium chloride precipitation method. Poly A$^+$ mRNA was prepared by oligo-dT cellulose chromatography. Oligo-dT-primed cDNA was prepared and cloned into λgtll.

Two partially overlapping deoxyoligonucleotides representing complementary strands of scu-PA cDNA were chemically synthesized. These two strands 5'-GGGCAGGTGTGCGCAGCCATCCCGGACT-3' and 5'-GGGCAGGCAGATGGTCTGTATAGTCCGGG-3' were annealed and filled in as described in the previous section, to produce a 49 nucleotide long probe having a specific activity of ~$10^8$ cpm/$\mu$g. This probe spans nucleotide 931 through 979 of the u-PA cDNA sequence (W.E. Holmes et al, Biotechnology 3, 923-929, 1985). Plaque hybridization was performed as described in the previous section. A second specifically primed cDNA library was prepared in λgtll, using the latter mentioned deoxyoligonucleotide to initiate first strand cDNA synthesis. Three overlapping cDNA clones were spliced together in pUC18 to produce a contiguous cDNA sequence encoding full length scu-PA (pULscu-PA) which was sequenced as described in the previous section.

A HindIII restriction endonuclease fragment from pULscu-PA, containing the complete coding sequence for scu-PA, 89 nucleotides of 5'-untranslated sequence and 78 nucleotides of 3'-untranslated sequence was inserted into the unique HindIII restriction endonuclease site of pSV328DHFR (Nelles et al. J. Biol. Chem. 262, 5682-5689, 1987) to yield pSVscu-PA-DHFR which was used for expression of scu-PA in CHO cells as described below.

I.3.Construction of t-PA/u-PA chimeric cDNA clones

For the construction of the t-PA/u-PA-s cDNA, the BlgII-Sau3AI fragment from pULscu-PA (nucleotides 399 to 732, encoding amino acids Arg[88] through Val $\overline{199}$) was ligated into BamHI digested M13mp18 to yield M13intl. The SstI fragment of pULt-PA (nucleotides -20 to 1421) encoding the amino acids from the $NH_2$-terminal Met$^{-35}$ through Leu[411] was then ligated into the SstI site of M13intl, upstream of the scu-PA sequence to yield M13t-PA-scu-PA. In this DNA molecule, both the t-PA and scu-PA sequences are in the same orientation. Splicing of the DNA at t-PA codon Thr[263] and scu-PA codon Leu[144] was accomplished by in vitro site directed mutagenesis using the single stranded template DNA of M13t-PA-scu-PA and the synthetic deoxyoligonucleotide deletion primer 5'-CTGAAATTTTAAGGTGGAGCAGGA-3' (New England Biolabs), which is complementary to the codons for amino acids Ser[260] to Thr[263] of t-PA and for the amino acids Leu[144] to Gln[147] of scu-PA. 200 ng of the phosphorylated deoxyoligonucleotide was heated with 1$\mu$g of the template at 65°C in 50 mM Tris-HCl buffer pH 7.8, 10 mM $MgCl_2$ and 20 mM DTT for 5 minutes and annealed by stepwise cooling to room temperature and 0°C. ATP was added to a concentration of 0.4 mM and dNTPs to a concentration of 0.05 mM in a final volume of 50 $\mu$l. 400 U $T_4$ DNA ligase and 5 units Klenow fragment of E.coli DNA polymerase I were added. The DNA obtained after 1 hr incubation at 14°C was used to transfect E.coli JM 101 cells.

250 ng of the deletion primer was radiolabeled to a specific activity of $10^8$ cpm/$\mu$g by incubation in 70 mM Tris-HCl buffer, pH 8.0, containing 10 mM $MgCl_2$ and 5 mM dithiotreitol, of 100 $\mu$Ci of $(\gamma^{-32P})$ATP and 10 units of $T_4$-polynucleotide kinase in a 20 $\mu$l reaction mixture, for 30 minutes at 37°C. This labeled probe was then used for plaque hybridization to identify the spliced DNA of M13t-PA/u-PA-s. Hybridization proceeded overnight at 22°C in 0.09 M Tris-HCl buffer pH 7.5, containing 0.9 M NaCl, 0.5 % Nonidet P40 detergent, 1X Denhardts, 6 mM EDTA, 1 mM Na-pyrophosphate, 0.1 mM ATP and 0.2 mg/ml E.coli tRNA. Nitrocellulose filters were washed for 1 hr in 75 mM NaCl at 49°C with several changes and exposed overnight to X-ray films. Plaques revealing a positive hybridization signal were selected for dideoxynucleotide sequencing in order to verify the deletion of undesired DNA.

For the construction of the t-PA/u-PA-e cDNA, the single stranded DNA from M13t-PA/scu-PA was used to replace the codon for Cys[264] (TGC) in the t-PA cDNA by a codon for Gly (GGA). Therefore, the deoxyoligonucleotide 5'-TGTCTCAGGCCTCCGGTGGAGCAG-3', complementary to nucleotides 969 through 992 of the t-PA cDNA sequence, was used for site-directed substitution mutagenesis and for plaque hybridization essentially as described in the previous paragraph. The resulting mutagenized M13 phage DNA was sequenced and used for site directed deletion mutagenesis with the deoxyoligonucleotide 5'-TGGAGGAGAGGAAAACTGAGGCTG-3' complementary to the codons for Gln[271] through Phe[274] (nucleotides 1000-1011) of t-PA and the codons for Ser[138] through Pro[141] (nucleotide 548-559) of scu-PA, as described above. Plaques containing the correctly spliced cDNA (M13t-PA/u-PA-e) were verified by DNA sequencing.

Both t-PA/u-PA-s and t-PA/u-PA-e cDNAs were reconstructed as follows: BglII-partial EcoRI restriction endonuclease fragments from M13t-PA/u-PA-s encoding Ser[1] through Thr[263] of the t-PA sequence and Leu[144] through Glu[163] of the scu-PA sequence, or from M13t-PA/u-PA-e, encoding Ser[1] through Phe[274] of the t-PA sequence and Ser[138] to Glu[163] of the scu-PA sequence, were isolated. These fragments were combined with a partial EcoRI-SstI restriction endonuclease fragment of pULscu-PA, encoding Phe[164] through Leu[411] and containing another 78 nucleotides of 3'-untranslated scu-PA cDNA sequence plus a remnant of pUC18 polylinker, and ligated in BglII-SstI digested pULscu-PA.

Expression vectors for t-PA/u-PA-s and t-PA/u-PA-e were constructed as follows. A HindIII-BglII restriction endonuclease fragment from pULt-PA encoding the signal sequence of t-PA and a BglII-SstI restriction endonuclease fragment encoding the mature t-PA/u-PA-s were combined and ligated in HindIII-SstI linearized pSV328DHFR (L. Nelles et al., J. Biol. Chem. 262, 5682-5689, 1987) to yield pSVt-PA/u-PA-s-DHFR. For the reconstruction of the complete t-PA/u-PA-e cDNA the HindIII-BglII restriction endonuclease fragment from pULt-PA encoding the signal sequence and a BglII-SstI restriction endonuclease fragment encoding the mature t-PA/u-PA-e were combined and ligated in HindIII-SstI linearized pUC18. From this construct the whole t-PA/u-PA-e cDNA was transferred as a HindIII restriction fragment to pSV328DHFR to yield pSVt-PA/u-PA-e-DHFR. The DNA sequence encoding a protein of 548 amino acids was confirmed by dideoxynucleotide sequencing and is represented in Figure 1.

## I.4. Eukaryotic expression of t-PA/u-PA chimeric cDNA clones

5$\mu$g of the pSVt-PA-DHFR, the pSVscu-PA-DHFR, the pSVt-PA/u-PA-s-DHFR, or the pSVt-PA/u-PA-e-DHFR plasmid were used to transfect DHFR-deficient Chinese Hamster Ovary cells. These cells were obtained from Dr. W. Fiers of the University of Ghent, Belgium. The cells were transfected using the calcium phosphate coprecipitation method (Graham and Van der Eb, Virology 52, 456-467, 1973). DHFR$^+$

cells were isolated by selection in $F_{12}$-medium (Gibco/BRL, Ghent, Belgium) depleted in glycine, hypoxanthine and thymidine, supplemented with 10% dialyzed fetal calf serum and were monitored for secretion of t-PA-related antigen or of low molecular weight in-PA-related antigen using specific ELISA assays (Holvoet et al., Thromb. Haemost. 54, 684-687, 1985; Stump et al., J. Biol. chem. 261, 17120-17126, 1986).

Large scale production was carried out, in which conditioned medium was harvested three times after consecutive 2 day incubation periods in serum free medium.

## I.5. Purification of t-PA/u-PA chimeric proteins from conditioned cell culture media

The chimeric proteins, t-PA/u-PA-s and t-PA/u-PA-e, were purified by chromatography on Zinc chelate-Sepharose followed by immunoadsorption on an insolubilized murine monoclonal antibody. Conditioned medium (batches of about 20 liters containing 430 ± 220 $\mu$g/liter of u-PA-related antigen for t-PA/u-PA-s, or batches of 25 to 35 liters containing about 1 mg/liter of u-PA-related antigen for t-PA/u-PA-e) were applied on a 5 x 25 cm column of Zinc chelate-Sepharose, previously equilibrated in 0.3 M NaCl, 0.02 M Tris-HCl buffer, pH 7.5, containing 0.01% Tween 80 and 10 KIU/ml aprotinin, at a flow rate of 250 ml per hour at 4°C. The column was washed with equilibration buffer and eluted with buffer containing 50 mM imidazole. Fractions of 7.5 ml were collected into tubes containing 2.5 ml 0.4 M arginine, 0.08 M citrate buffer, pH 5.0, and 40 KIU/ml of aprotinin. The u-PA-related antigen as measured by ELISA, eluted in a peak which virtually coincided with the main protein peak. The purification step with Zinc chelate-Sepharose yielded a nearly quantitative recovery (≥85%) and a reduction in volume of 75- to 100-fold.

The pooled u-PA containing fractions of the Zinc chelate-Sepharose column were chromatographed at 4°C at a flow rate of about 15 ml per hour on a 1.6 x 20 cm immunoadsorption column containing an insolubilized monoclonal antibody against t-PA (MA-1C8) for t-PA/u-PA-s or against u-PA (MA-4D1E8) for t-PA/u-PA-e. The column was washed with 0.3 M NaCl, 0.02 M Tris-HCl buffer, pH 7.5, containing 0.01% Tween 80 and 10 KIU/ml aprotinin and then eluted with 2 M KSCN in the same buffer. Fractions of 7.5 ml were collected into tubes containing 2.5 ml 0.4 M arginine, 0.08 M citrate buffer, pH 5.0, and 40 KIU/ml of aprotinin. The fractions containing u-PA-related antigen were pooled and dialyzed against 0.3 M NaCl, 0.1 M arginine, 0.02 M citrate buffer, pH 5.0, containing 0.01 % Tween 80 and 10 KIU/ml aprotinin.

After immunoadsorption chromatography, the overall yield of u-PA-related antigen was about 80 % and approximately 330 or 800 $\mu$g purified material was obtained per liter of conditioned medium for t-PA/in-PA-s or t-PA/u-PA-e respectively. With 10 KIU/ml aprotinin added to the cell culture media and the buffers used during purification, the chimeric proteins were obtained nearly exclusively in the single chain form. Before use, samples of the chimeric proteins were chromatographed on benzamidine-Sepharose and fractions devoid of amidolytic activity against S-2444 were pooled. Aprotinin was efficiently removed by extensive washing on a Centricon 30 microconcetrator (Amicon) as confirmed by titration with plasmin.

## Example II

## Production of variants to t-PA/u-PA-e

Three variants of the chimeric plasminogen activator t-PA/in-PA-e were produced by culturing eukariotic cells transformed by means of recombinant DNA technology. The production process comprised the following steps:

II.1. Construction of cDNA encoding t-PA-$\Delta K_1$V$K_2$/u-PA (see also Nelles et al. Thromb. Haemost., in press)

For easier manipulation of the t-PA cDNA during the reconstruction of the t-PA/u-PA variant chimeras, most of its 5'-untranslated region was removed as follows: from pULt-PA, an 850 bp EcoRI restriction fragment containing the 5'-untranslated sequence and the $NH_2$-terminal codons was cloned in pUC18 and truncated at its 5'-end by consecutive exonuclease III and S1 nuclease action as described by Henikoff (Gene 28, 351-359, 1984). The resulting plasmid, called pt-PA-sh-5', which has a HindIII restriction site 9 bp upstream of the start codon for t-PA and which contains nucleotides 76 to 801 of the t-PA cDNA sequence was used for the construction of pt-PA-$\Delta K_1$∇$K_2$/u-PA as described below. The kringle 2 region in the cDNA of the chimera t-PA/u-PA-e (Lijnen et al., J. Biol. Chem. 263, 19083-19091, 1988) was duplicated in the following way. A BlgII-Ball restriction fragment of pt-PA/u-PA-e, spanning nucleotide 189 of the t-PA cDNA to nucleotide 582 of the scu-PA cDNA and which contains the complete coding region for the t-PA A-chain, was made blunt and inserted into the unique Ball site of pt-PA/u-PA-e to give pInt1. pInt1 thus contains a tandem repeat of the sequences coding for the entire t-PA A-chain, one of which is inserted in the region coding for the u-PA B-chain. From this plasmid, restriction endonuclease fragments were used for oligonucleotide directed deletion mutagenesis in the following way: from pInt1, an 859 bp EcoRI restriction fragment covering nucleotides 802 to 1011 of the t-PA cDNA, nucleotide 548 to 582 of the u-PA cDNA and again nucleotides 188 to 801 of the t-PA cDNA was cloned into the EcoRI site of M13mp19, yielding m859Int1. Deletion mutagenesis with the deoxyoligonucleotide 5'-GTTTCCCTCAGAGCAGGAGGGCAC-3' was performed as described to fuse the codon for $Cys^{261}$ to the codon for $Ser^{174}$ of t-PA, giving m859Int2 in which the 3'-end of the cDNA coding for $K_2$ was spliced to the 5'-end of the interkringle sequence. A 614 bp BlgII-EcoRI restriction fragment from pInt1 containing the F, E and $K_1$ domains and part of the $K_2$ domain, was cloned into BamHI-EcoRI linearized M13mp19, which yielded m614Int1. Deletion mutagenesis with the deoxyoligonucleotide 5'-CCCAAAGTAGCACGTGGCCCTGGT-3', fusing the $Thr^{91}$ codon to the $Cys^{180}$ codon, yielded m614Int2, with deletion of the complete kringle 1 and the interkringle sequences of t-PA.

For the reconstruction of the complete t-PA-$\Delta K_1 \nabla K_2$/u-PA cDNA, the PstI-EcoRI restriction fragment from pt-PA-sh-5' containing nucleotides 209 to 801 of t-PA was replaced by the corresponding PstI-EcoRI fragment of m614Int2 yielding pInt2. Then, the EcoRI restriction fragment of m859Int2, carrying the t-PA sequences coding for $Asn^{205}$ to $Cys^{261}$ followed by the codons for $Ser^{174}$ to $Trp^{204}$, was cloned in the EcoRI site of pInt2 to give pInt3. Finally, a HindIII-partial EcoRI restriction fragment from pInt3 carrying the t-PA sequences, including the start codon, the F and E domain, the first copy of $K_2$ and part of the second copy of $K_2$, was combined with a partial EcoRI-SstI restriction fragment of pt-PA/u-PA, spanning the remaining parts of $K_2$ and the complete u-PA B-chain sequences, and inserted in HindIII-SstI linearized pUC18 to yield pt-PA-$\Delta K_1 \nabla K_2$/u-PA.

II.2.Construction of cDNA encoding t-PA-$\Delta FE$/u-PA

The deletion of the F and E domains of t-PA was performed on a restriction endonuclease fragment from pt-PA-sh-5' containing the complete signal sequence and the coding sequence for the F, E and $K_1$ domain and part of the $K_2$ domain in the following way: from the plasmid pt-PA-sh-5', the 732 bp HindIII-EcoRI fragment containing the t-PA A-chain sequences was transferred to M13mp19 to yield m732Int1. This plasmid was used for deletion mutagenesis with the deoxyoligonincleotide 5'-GGCCCTGGTATCTTGGTAAGATCT-3' to delete the F and E domains. After mutagenesis the shortened HindIII-EcoRI restriction fragment was combined with the partial EcoRI-SstI fragment of pt-PA/u-PA containing the remaining parts of the t-PA A-chain and the u-PA B-chain and ligated in HindIII-SstI linearized pUC18 to yield pt-PA-$\Delta FE$/u-PA.

II.3.Construction of cDNA t-PA-$\Delta FEK_1 \nabla K_2$/u-PA

To delete the F and E domains from t-PA-$\Delta K_1 \nabla K_2$/u-PA, an intermediairy plasmid of the pt-PA-$\Delta K_1 \nabla K_2$/u-PA construction was used. From pInt2, described in section II.1., a 468 bp HindIII-EcoRI restriction fragment containing the start codon of the t-PA cDNA and downstream sequences coding for the F and E domains and part of the first $K_2$ copy, was cloned in the HindIII-EcoRI restriction sites of M13mp19 giving m468Int1 (not shown). This DNA was used for deletion mutagenesis with the deoxyoligonucleotide described in section II.2, to delete the F and E domains. This yielded m468Int2$\Delta FE$. The reconstruction of the complete cDNA of the deletion mutant of t-PA-$\Delta K_1 \nabla K_2$/u-PA involved the insertion of the EcoRI restriction fragment of m859Int2, encoding t-PA codons $Asn^{205}$ to $Cys^{261}$ followed by $Ser^{174}$ to $Trp^{204}$, into the EcoRI restriction site of m468Int2$\Delta FE$, resulting in m468Int3$\Delta FE$. A HindIII-partial EcoRI restriction fragment from m468Int3$\Delta FE$, containing most of the t-PA A-chain sequences, was then combined with the partial EcoRI-SstI restriction fragment of pt-PA/u-PA mentioned earlier, and ligated in HindIII-SstI linearized pUC18 to yield pt-PA-$\Delta K_1 \nabla K_2$/u-PA.

II.4.Construction of plasmid pCM$\beta$Neo for expression of t-PA/u-PA-e variant chimeras

The expression plasmid pCM$\beta$Neo was constructed using pUC18 in order to obtain a high expression level vector that can be multiplied in E.coli as a high copy number plasmid. This vector drives transcription via the human cytomegalovirus immediate-early promoter, while the selection marker, neomycin-phosphotransferase, is controlled by the SV40 early promoter. The vector has a single HindIII restriction site for insertion of cDNA's. A HindIII-BamHI restriction fragment containing the neomycine phosphotransferase gene from pNeo (Pharmacia, Uppsala, Sweden) was ligated into BglII-HindIII linearized pSV328A$^+$ (Van Heuvel et al., J. Gen. Virol. 67, 2215-2222, 1986), resulting in pSV328Neo from which the HindIII site was destroyed. From the plasmid pSV328DHFR, a PvuII-NdeI restriction fragment, containing the early promoter/enhancer from SV40, the multiple cloning site and the rabbit $\beta$-globin transcription termination signal, was cloned into NdeI-SspI linearized pUC18 to yield pSV18. This plasmid was opened with PvuII, thereby removing the multiple cloning site of pUC18 and most of the $\beta$-galactosidase sequences. The PvuII-NdeI restriction fragment of pSV328Neo containing the SV40 early promotor, the neomycine gene and the rabbit $\beta$-globin transcription termination signals, with a blunted protruding end, was inserted to produce pSV$\beta$Neo. In this plasmid the small ScaI-HindIII restriction fragment, containing an SV40 early promoter/enhancer, was replaced by the small Scal-HindIII restriction fragment from pUC18, yielding pUCβNeo. Finally, two restriction fragments of the plasmid pRR23 which together form the major part of the human cytomegalovirus immediate early promotor/enhancer (Boshart et al., Cell 41,521-530, 1985), were isolated. A HindIII-NdeI restriction fragment, of which the HindIII site was blunted, and a NdeI-Sau3A1 restriction fragment, of which the Sau3A1 end was blunted and HindIII linkered, were ligated in HindIII-SspI linearized pUCβNeo to yield pCMβNeo.

II.5.Expression of the t-PA/u-PA-e variant chimeras

Expression plasmids for cDNA encoding t-PA-$\Delta K_1 \nabla K_2$/u-PA-e, t-PA-$\Delta FE$/u-PA-e and t-PA-$\Delta K_1 \nabla K_2$/u-PA-e were constructed by inserting the HindIII restriction fragments from t-PA-$\Delta K_1 \nabla K_2$/u-PA, pt-PA-$\Delta FE$/u-PA and pt-PA-$\Delta FEK_1 \nabla K_2$/u-PA, each containing the complete coding sequence of the respective variant chimer, into the unique HindIII restriction site of pCMβNeo, to yield pCMt-PA-$\Delta K_1 \nabla K_2$/u-PA, pCMt-PA-$\Delta FE$/u-PA and pCMt-PA-$\Delta FEK_1 \nabla K_2$/u-PA respectively.

The expression plasmids containing the mutant cDNA's were used to stably transfect Chinese hamster ovary cells as described in section I.4. Transfected cells were selected for resistance to 400 μg/ml geneticin (Gibco/BRL), resistant colonies were screened for t-PA-related antigen secretion, and suitable cell lines were chosen for large scale production of chimeric proteins. Large scale production was carried out essentially as described before in Optimem medium (Gibco/BRL) containing 100 IU/ml Penicilline, 100 μg/ml streptomycin and 10 KIU/ml aprotinin.

II.6.Purification of t-PA/u-PA-e variant chimeric proteins from conditioned cell culture media

The recombinant t-PA/u-PA-e moieties, characterized in the present study were purified by chromatography of batches of 15 to 40 liters of conditioned medium on Zinc chelate-Sepharose, essentially as described above, followed by immunoadsorption on an insolubilized murine monoclonal antibody against u-PA (MA-4D1E8) (for t-PA/u-PA-e and t-PA-$\Delta K_1 \nabla K_2$/u-PA-e) or against t-PA (MA-1C8) (for t-PA-$\Delta FEK_1 \nabla K_2$/u-PA-e). t-PA-$\Delta FE$/u-PA-e was purified by chromatography on SP-Sephadex C50, essentially as previously described (Stump et al., J. Biol. Chem. 261, 1274-1278, 1986) followed by chromatography on insolubilized MA-1C8. Elution from the antibody column was performed with 1.6 M KSCN and the fractions containing t-PA-related antigen were pooled and dialyzed against 0.3 M NaCl, 0.1 M arginine, 0.02 M Tris-HCl buffer, pH 7.4, containing 0.01% Tween 80 and 10 KIU/ml aprotinin. The mean yields of t-PA-related antigen (2 preparations) were 0.70 mg/l for t-PA/u-PA-e, 0.46 mg/l for t-PA-$\Delta K_1 \nabla K_2$/u-PA-e, 0.28 mg/l for t-PA-$\Delta FE$/u-PA-e and 0.32 mg/l for t-PA-$\Delta FEK_1 \nabla K_2$/u-PA-e. Two-chain derivatives in the preparations were inhibited by addition of Glu-Gly-Arg-$CH_2$Cl (final concentration $10^{-4}$ M). Before use, aprotinin and Glu-Gly-Arg-$CH_2$Cl were removed by extensive washing on a Centricon 30 microconcentrator (Amicon).

Example III

Biochemical characterization

The two chimeric plasminogen activators produced in example I and the three variants thereof produced in example II were subjected to various tests for biochemical characterization. Four other plasminogen activators, namely home-made u-PA, commercial u-PA, home-made t-PA and commercial t-PA, were used for comparative purposes.

The home-made u-PA was a recombinant single-chain u-PA produced by expression in Chinese Hamster Ovary cells and purified by chromatography on zinc chelate-Sepharose and chromatography on SP-Sephadex C50 (compare Stump et al, J. Biol. Chem., 261, 1274-1278, 1986, which is incorporated herein by way of reference). The two-chain form thereof was produced by plasmin activation of the single-chain form.

Commercial u-PA as used (Saruplase) was a recombinant u-PA produced by expression in E. coli cells and provided by Grünental GmbH of Aachen, FRG. This product was in single-chain form, and a two-chain form was produced from it by plasmin activation.

Home-made t-PA was produced by expression of t-PA cDNA in Chinese Hamster Ovary cells and purified from a conditioned culture medium by a combination of chromatography on zinc chelate-Sepharose and immunoadsorption on insolubilized monoclonal antibody MA-1C8, in the same way as described in examples I and II.

Commercial t-PA (Activase) was a recombinant t-PA provided by Genentec Inc., South San Francisco, USA.

As a first step in biological characterisation, the following assays were used:

III.1. SDS-PAGE, i.e. electrophoresis in sodium dodecyl sulphate-polyacrylamide gel, used to determine molecular weights. In this assay, 12% slab gels were used, compare Lemmli et al, Nature, 227, 680-685, 1970, which is incorporated herein by way of reference.

III.2. Immuno blotting of SDS-PAGE results with antisera against u-PA and t-PA. This was performed on nitrocellulose sheets according to Towbin et al, Proc. Natl. Acad. Sci. USA 76, 4350-4354, 1979, which is incorporated herein by way of reference.

III.3. Amino acid analysis. This was performed on a Beckman 119 CL amino acid analyser after 20 hours of hydrolysis in 6 M HCl at 110°C, in vacuo.

III.4. Specific activity. This was performed on fibrin plates according to Astrup et al, Arch. Biochem. Biophys. 40, 346-351, 1952, which is incorporated herein by way of reference. The results were as follows:

Purified single-chain t-PA/u-PA-s (t-PA/scu-PA-s) migrated as a doublet band under non-reducing conditions on SDS-PAGE, and as a single main band with $M_r$ approximately 70,000 under reducing conditions. Both bands of the doublet seen on unreduced gel are immunologically active with antisera against both u-PA and t-PA. Purified single-chain t-PA/u-PA-e (t-PA/scu-PA-e) migrated as a single main band on SDS-PAGE, with $M_r$ about 70,000 under reducing conditions.

The amino acid compositions of both chimeric proteins were in agreement with those derived from the known sequences of the t-PA/u-PA molecules. The specific activities on fibrin plates were initially found as 75,000 IU/mg for t-PA/scu-PA-s and 65,000 IU/mg for t-PA/scu-PA-e, and 71,000 IU/mg for t-PA/tcu-PA-s and 68,000 IU/mg for t-PA/tcu-PA-e, as determined by comparison with the International Reference Preparation for Urokinase obtained from The National Institute for Biological Standards and Control, London UK . In a subsequent study, higher specific activities were obtained for t-PA/scu-PA-e and for t-PA/tcu-PA-e. These values and the corresponding specific activities for home-made t-PA, Saruplase, home-made tcu-PA and Activase, are represented in table I.

The three variants to t-PA/u-PA-e were obtained mainly in the single-chain form as shown by SDS-PAGE after reduction with DTE. Immunoblotting confirmed the presence of both t-PA-related and u-PA-related antigen in the chimeras. The specific activity, measured with S-2444 (a chromogenic substrate, Pyroglu-Gly-Arg-pNA) of all single chain variants was less than 1,000 IU/mg. The amino acid compositions of the three variants were in agreement with their amino acid sequence, as deduced from their c-DNA sequence. The specific activities on fibrin plates ranged between 68,000 IU/mg and 200,000 IU/mg and have been indicated in table I.

Further, the following tests were used:

III.5. Treatment of the chimeras with plasmin.

The chimeric proteins of example I (final concentration 1.0-2.0 $\mu$M) in 0.02 M Tris-HCl buffer, pH 7.4, containing 0.3 M NaCl, 0.2 M arginine and 0.01% Tween 80, were treated with plasmin (final concentration range 0.25 to 8% on a molar basis) at 37°C. At timed intervals (0 to 30 minutes), 5 $\mu$l were removed and added to 800 $\mu$l of the same buffer containing S-2444 at a final concentration of 0.3 mM. Urokinase activity was determined from measurements of the absorption at 405 nm and expressed in IU by comparison with the International Reference Preparation for Urokinase. The specific activities of t-PA/scu-PA-s and t-PA/scu-PA-e were 320 IU/mg and 1000 IU/mg resp. Plasmin caused a time-dependent conversion of the single-chain proteins to amidolytically active urokinase. Maximum conversion resulted in an amidolytic activity corresponding to a specific activity of about 43,000 IU/mg for t-PA/scu-PA-s and 50,000 IU/mg for t-PA/scu-PA-e (100,000 ± 6,900 in a subsequent study). The molecular form of the chimera at the end of the treatment was monitored on 12% SDS-PAGE after reduction. This showed complete conversion of the single-chain chimeras to two-chain molecules following incubation with plasmin (3% on a molar basis) for 30 minutes at 37°C. Corresponding values for home-made tcu-PA and plasmin-treated Saruplase have been given in table I.

The variant chimeras of Example II (final concentration 5-10 $\mu$M) in 0.05 M Tris-HCl buffer, pH 7.4, containing 0.038 M NaCl, 0.01% Tween 80 and 0.1 M Arginine, were treated with plasmin (1-3% on a molar basis) for 30 minutes at 37°C. Generation of urokinase activity was monitored with S-2444 (final concentration 0.3 mM) by measurement of the adsorbance at 405 nm after at least 200-fold dilution of the samples. Again, plasmin caused a time-dependent conversion of all t-PA/scu-PA-e variants to amidolytically active t-PA/tcu-PA-e derivatives. The specific amidolytic activities after maximum conversion have been given in table I. SDS-PAGE after reduction of the plasmin-treated variants with DTE, revealed the presence of two polypeptide bands, one band migrating with a $M_r$ compatible with the COOH-terminal polypeptide chain of u-PA and another band migrating with a $M_r$ compatible with the expected size of the fragment of the NH$_2$-terminal chain of t-PA. This identification was confirmed by immunoblotting of reduced SDS-PAGE. To obtain a stock of the two-chain derivates, plasmin was neutralised when a maximum activation was obtained, by addition of excess aprotinin (2,000 KIU/ml) and the samples were applied to benzamidine-Sepharose. Elution was performed with 0.15 M NaCl, 0.1 M Glycine-HCl buffer, pH 2.2, and 1 ml fractions were collected directly into tubes containing 80 $\mu$l of 1 M Tris-HCl buffer, pH 9.0. Fractions with amidolytic activity against S-2444 were pooled and used for further analysis.

III.6. Activation of plasminogen.

Activation of plasminogen (final concentration 10 to 95 $\mu$M) by the two-chain form of the chimeras of example I (final concentration 2.5 to 5 nM) was measured by incubation of plasminogen at 37°C in 0.05 M Tris-HCl buffer of pH 7.4, containing 0.038 M NaCl and 0.01% Tween 80. The generated plasmin at different time intervals (0 to 6 minutes) was measured with chromogenic plasmin substrate S-2251 (final concentration 0.3 mM) after 100- to 200-fold dilution of the sample. Initial activation rates were obtained from plots of the concentration of generated plasmin versus incubation time. From these plots, the kinetic constants $K_m$ and $k_2$ were obtained. The catalytic efficiencies ($k_2/K_m$) have been given in table I.

With regard to the variants of example II, activation of native plasminogen (final concentration 15 to 75 $\mu$M) by the two-chain t-PA/u-PA-e variants (final concentration 1.0-1.5 nM) was measured by incubation of plasminogen at 37°C in 0.05 M Tris-HCl buffer of pH 7.4, containing 0.038 M NaCl and 0.01 % Tween 80. The generated plasmin at different time intervals (0 to 5 minutes) was measured with S-2251 (final concentration 1 mM) after 50-fold dilution of the sample. Initial activation rates were obtained from plots of the concentration of generated plasmin versus incubation time. The kinetic constants were determined by linear regression analysis on Lineweaver-Burk plots of the data. The catalytic efficiencies ($k_2/K_m$) are given in table I.

The effect of fibrin on the activation rate of plasminogen by the two-chain chimeras of example I was evaluated by incubation of plasminogen (final concentration 0.1 to 2 $\mu$M) at 37°C in 0.05 M Tris-HCl buffer, pH 7.4, containing 0.038 M NaCl and 0.01% Tween 80 with CNBr-digested fibrinogen (final concentration 0.05 to 1.0 $\mu$M) prior to addition of the chimeras (final concentration 1.25 or 1.5 nM). The plasmin generated was measured as described above following 20-fold dilution of the sample. Addition of CNBR-digested fibrinogen resulted in a concentration-dependent increase of the activation rate of plasminogen by both chimeras. The stimulation factor of 2- to 3-fold is very similar to that observed with home-made tcu-PA or plasmin treated Saruplase but much lower than the 140- to 200-fold observed with t-PA (table I).

The effect of fibrin on the activation rate of plasminogen by the two-chain forms of the variants of example II was evaluated by incubation of plasminogen (final concentration 1 $\mu$M) at 37°C in 0.05 M Tris-HCl buffer, pH 7.4, containing 0.038 M NaCl and 0.01% Tween 80 with CNBr-digested fibrinogen (final concentration 0.1-2.0 $\mu$M) prior to addition of the variant (final concentration 1 nM). The plasmin generated was measured as described above after 20-fold dilution of the sample, and initial activation rates were determined. Addition of CNBr-digested fibrinogen resulted in a concentration-dependent stimulation of the activation rate of plasminogen by all two-chain forms of the three variants. Table I shows the stimulation factor, calculated as the ratio of the initial activation rate in the presence of infinite fibrin concentration over that in the absence of fibrin. This stimulation factor is of the same order as that of tcu-PA.

III.7. Binding to purified fibrin.

Human fibrinogen (final concentration 0 to 3.2 mg/ml) in 0.038 M NaCl, 0.05 M Tris-HCl buffer, pH 7.4, containing 0.01% Tween 80 and 1 mg/ml BSA, was mixed with scu-PA, tcu-PA, t-PA/scu-PA-s, t-PA/tcu-PA-s, t-PA/scu-PA-e, t-PA/tcu-PA-e, single-chain or two-chain t-PA or Activase (final concentration 50 to 200 ng/ml). The mixture was clotted by addition of thrombin to a final concentration of 20 NIH units/ml. Following a 1-minute incubation at 37°C, thrombin was inactivated by the addition of D-Ile-Pro-Arg-CH$_2$Cl (10 $\mu$M final concentration) to prevent inactivation of urokinase and the samples were centrifuged for one minute at 10,000 g. The concentrations of u-PA-related or t-PA-related antigen in the supernatants were determined by specific ELISAS. Compare Stump et al, J. Biol. Chem. 261, 17120-17126, 1986, and Holvoet et al, Thromb. Haemostas. 54, 684-687, 1985, which are incorporated herein by way of reference.

For the variants of Example II, plasminogen-depleted human fibrinogen (final concentration 0 to 3.2 mg/ml) in 0.038 M NaCl, 0.05 M Tris-HCl buffer, pH 7.4, containing 0.01% Tween 80 and 1 mg/ml bovine serum albumin was mixed with the single-chain or two-chain t-PA/u-PA-e variants (final concentration 100 ng/ml). The mixture was clotted by addition of thrombin to a final concentration of 20 NIH units/ml. Following a 1-minute incubation at 37°C, thrombin was inactivated by the addition of D-Ile-Pro-Arg-CH$_2$Cl (final concentration 10 $\mu$M) and the samples were centrifuged for one minute at 10,000 g. The concentration of t-PA-related antigen in the supernatants was determined by specific EllSA as above.

The results are given in table I. It appears that both single-chain and two-chain chimeric proteins have a fibrin-affinity intermediate that of u-PA and t-PA.

In table I, all values for specific activity on fibrin plates and on S-2444 are mean values ± SD of three or more determinations. The values for fibrin-affinity are mean values ± SD of three to seven experiments. The asterisk mean that a single-chain form was used.

III.8. Fibrin clot lysis and fibrinogenolysis in a plasma milieu

The relative fibrinolytic potency and fibrin specificity of the chimeric proteins of examples I and II and of the comparative substances (final concentration range 0.01-5.0 µg/ml) were measured in a system composed of a $^{125}$I-fibrin-labelled human plasma clot, suspended in human citrated plasma. The test method has been described by Zamarron et al, Thromb. Haemost. 52, 19-23, 1984, which is incorporated herein by way of reference. Fibrinogen levels were measured with a routine coagulation rate assay.

The single-chain and two-chain forms of all plasminogen activators tested induced a time- and concentration-dependent lysis of the labelled plasma clot immersed in human plasma. The results were indicated as the concentration required to obtain 50% clot lysis in 2 hours ($C_{50}$) and as the residual fibrinogen level after 2 hours. These results are given in table II. It appears that the values are either comparable with those of u-PA or with those of t-PA.

Example IV

Pharmacokinetic properties in animal models

The pharmacokinetics of the plasminogen activators were determined by measurement of t-PA-related or u-PA-related antigen concentrations in plasma at different time intervals after intravenous bolus injections of 0.1 mg/kg of test material in groups of 3 rabbits. Compare Holvoet et al, Thromb. Haemost. 54, 684-687, 1985 and Stump et al, J. Biol. Chem. 261, 17120-17126, 1986, which are incorporated herein by way of reference. The results were plotted on semilogarithmic paper and fitted with a sum of two exponential terms, $C(t) = Ae^{-\alpha t} + Be^{-\beta t}$ by graphical curve peeling (Gibaldi and Perrier, Pharmacokinetics, Marcel Dekker Inc. New York, 1983 which is incoporated herein by way of reference). Thereto, the linear terminal portion of the antigen versus time curves were extrapolated to yield the ordinate intercept B. This line had a slope -$\beta$. The extrapolated values were subtracted from the initial values and the corrected data were fitted with a line which had a slope - $\alpha$ and an ordinate intercept A. The disappearance of antigen from plasma was represented by a two-compartment mammillary model composed of one central and one peripheral compartment with elimination occurring from the central compartment. Pharmacokinetic parameters were calculated from these coefficients and exponents using standard formulas derived by Gibaldi and Perrier. The following drug disposition parameters were derived: volume of the central compartment $V_c$ = dose/(A + B); total volume of distribution $V_D$ = dose/B; extrapolated area under the curve AUC = A/$\alpha$ + B/$\beta$; plasma clearance $Cl_p$ = dose/AUC; initial half life time $t^{1/}(\alpha)$; and terminal half-life time $t^{\frac{1}{2}}(\beta)$. A summary of the results of the most relevant parameters $t^{\frac{1}{2}}(\alpha)$ and $Cl_p$ has been given in table III.

The pharmacokinetic parameters of the disposition of chimeric proteins and comparative substances from blood after bolus injection were also determined in hamsters. The measurements and calculations were performed in the same way as with rabbits, following a bolus injection of 0.25 mg/kg in groups of 3 hamsters each. A summary of the results has been given in table III.

Further, a series of tests was performed with plasminogen activators (Saruplase and t-PA- ΔFE/scu-PA-e) in baboons. u-PA-related antigen was determined in bloodsamples taken before and 1, 2, 5, 7, 10, 15, 20 and 30 minutes after cessation of infusion of Saruplase, whereas t-PA-related antigen was determined in bloodsamples taken before and 15, 30, 60, 90 and 120 minutes after the end of the infusion of t-PA-Δ FE/scu-PA-e. The experimental data describing the disappearance of antigen from plasma were fitted with a sum of two exponential (exp) terms: $C(t) = R \exp(-\alpha t) + S \exp(-\beta t)$. The coefficients (R and S) and exponents ($\alpha$ and $\beta$) of this function were obtained from semilogarithmic plots by graphic curve peeling as described above. Pharmacokinetic parameters were calculated from these coefficients and exponents using standard formulas derived from Gibaldi and Perrier. The resulting values for the initial half-life time and plasma clearance are given in table III, where the data represent mean values ± SEM. The data indicated by an asterisk have p <0.01 versus Saruplase. The scu-PA tested in rabbits was a natural scu-PA obtained from Sandoz, Basel, Switserland. In the hamster model, a home-made scu-PA was used.

It can be derived from table III that the three chimeric proteins of example II have a longer initial half-life time and a significantly reduced clearance when compared with u-PA and t-PA. Such longer initial half-life time and delayed clearance are not shown by the chimeric proteins of example I.

Example V

Thrombolytic properties in animal models

The thrombolytic potency of the chimeric proteins and comparative substances was evaluated in rabbits, hamsters and baboons.

A rabbit jugular vein thrombosis model has been described earlier by Collen et al, J. Clin. Invest. 72, 368-376, 1983, which is incorporated herein by way of reference. In the present study, $^{125}$I-labeled jugular vein thrombi were aged for 30 minutes, and then the plasminogen activators were administered as a 10% bolus injection followed by continuous infusion of the remaining 90% dose over 4 hours via a contralateral marginal ear vein. Thrombolysis was quantitated 30 minutes after the end of the infusion by counting the residual radioactivity in the jugular vein segment. Blood samples of 1 ml were drawn into 0.01 M citrate and used for determinations of u-PA antigen or t-PA antigen and of residual fibrinogen and $\alpha_2$-antiplasmin levels.

In a first test series, lysis measured 30 minutes after the end of infusion of solvent into control animals was 11% $\pm$ 1%. A linear dose-dependent lysis with similar slope was obtained for all three compounds tested, namely natural scu-PA, t-PA/scu-PA-s and t-PA/scu-PA-e. A similar range of lysis was produced by all three compounds when infused over a concentration range of 0.2 to 3.2 mg/kg, which produced 12% to 34% lysis. The dose response data, after correction for background lysis of 11 $\pm$ 1%, were fitted with a linear regression line, forced through the origin, yielding correlation coefficients of $\geq$ 0.95. Lysis was expressed both as % lysis per mg/kg administered dose (thrombolytic potency) and as % lysis per $\mu$g/ml steady state u-PA-related or t-PA-related antigen (specific thrombolytic activity). The slopes of the lysis versus dose regression lines were very similar for all three compounds, whereas lysis per $\mu$g/ml plasma antigen level was 2-fold higher for the two chimeras than for natural scu-PA.

In a second series of experiments, the lysis measured 30 minutes after the end of infusion of solvent into control animals was 8 $\pm$ 1%. A linear dose-dependent degree of clot lysis was obtained with all three compounds tested, namely Saruplase, t-PA-$\Delta$FE/scu-PA-e and Activase. However, the slope obtained with t-PA-$\Delta$FE/scu-PA-e was significantly steeper than with Saruplase or with Activase. The data are represented in table IV.

All plasminogen activators tested produced thrombolysis in the absence of systemic fibrinolytic activation, as evidenced by virtually unchanged fibrinogen levels 30 minutes after the end of the infusion.

The thrombolytic potency of several plasminogen activators (chimeras, variants and comparative proteins) was further evaluated in a hamster pulmonary embolism model.

Outbred hamsters (Pfd Gold, University of Leuven, Belgium) 6 to 8 weeks old, with a body weight of 80 to 100 g, were anesthetized by intraperitoneal injection of 0.3 ml Pentobarbital diluted to 20 mg/ml in saline, which was administered 10 minutes after intramuscular injection of 50 $\mu$l of 0.25 mg/ml of atropin. A 2FG catheter was then placed in the femoral vein for blood sampling.

The procedure for producing a pulmonary embolus was as follows. A mixture of 300 $\mu$l fresh frozen human plasma, 5 $\mu$l of human $^{125}$I-labeled fibrinogen containing approximately 500,000 cpm of $125_I$ and 50 $\mu$l of a mixture of bovine thrombin (10 units/ml) and $CaCl_2$ (0.5 M) was aspirated into a 8FG catheter. The catheter was incubated for 30 minutes and 37°C, the plasma clot was then dislodged gently with positive pressure, washed for 30 minutes in saline, cut in 1 cm segments (total volume approximately 25 $\mu$l) and the radioisotope content was meaured. The radiolabeled clot was then aspirated into a 6FG catheter for injection. The left jugular vein was exposed and the catheter containing the labeled plasma clot was introduced and advanced for 1 cm into the brachiocephalic vein, where it was injected. The clot usually embolized into the lungs, but was occasionally trapped in the heart. The 6FG catheter was then replaced by a 2FG catheter, through which 0.1 ml of a 20 mg/ml sodium iodide solution was injected, followed by a bolus injection of 1,000 units/kg of heparin.

Thrombolytic agents or saline were infused over 60 minutes via the 2FG jugular vein catheter using a constant rate infusion pump, after dilution to a total volume of 350 $\mu$l with saline containing 0.01% Tween 80.

Thirty minutes after the end of the infusion, the animal was sacrificed with an overdose of Pentobarbital, the thorax was opened and the caval veins and aorta were clipped with a small hemoclip. The heart and both lungs were removed, separated from each other, and their radioisotope content was determined. The extent of clot lysis was determined as the difference between the radioactivity initially incorporated in the clot and the residual radioactivity in the lungs and the heart.

Blood samples (0.2 ml) were drawn into trisodium citrate (0.011 M final concentration), just before the start of the infusion, and 60 and 90 minutes later. These samples were used for measurement of radioactivity, fibrinogen, $\alpha_2$-antiplasmin and t-PA-related or u-PA-related antigen.

The infusions produced virtually linear dose-response curves, in the absence of systemic fibrinogen or $\alpha_2$-antiplasmin consumption. The mean values of the dose response data (% lysis versus dose in mg/kg for determination of thrombolytic potency or % lysis versus steady state plasma level in µg/ml for determination of specific thrombolytic activity) were corrected for background lysis, and the corrected data were fitted with a linear regression line forced through the origin, using a statistical program. The line slopes were expressed as % lysis per mg/kg of compound infused, or as % lysis per µg/ml steady state plasma level of t-PA-related or u-PA-related antigen. The data are represented in table IV, where it can be seen that most of the plasminogen activators tested produce similar results, except for t-PA-dhFE/scu-PA-e which shows significantly higher values for the thrombolytic potency.

The thrombolytic potency of some plasminogen activators (Saruplase and t-PA-ΔFE/scu-PA-e) was further evaluated in a baboon femoral vein thrombosis model. To that end, baboons (Papio hamadraeus) of either sex, weighing 7 to 18 kg, were anesthetized with 10 mg/kg ketamine hydrochloride (Imalgene 500, Sanofi, Brussels, Belgium) and 0.06 mg/kg atropine intramuscularly, after premedication with 5 mg diazepam intramuscularly. After endotracheal intubation, the lungs were ventilated with a respirator. Anesthesia was maintained with 30 mg pentobarbital intravenously.

The surgical procedure for femoral vein clot formation was as follows. A femoral vein was exposed through a 5 cm incision of the inguinal region and the vein was cleared from the entry of the femoral vein in the groin to 2 cm below the sapheno-femoral junction. Small side branches and the deep femoral vein were ligated, except for the predominant musculocutaneous branch, which was canulated with a Portex canulla. A woolen thread was then introduced in the lumen of the vein over a distance of 4 cm. When bleeding had ceased, the vein was clamped both proximally and distally to isolate a vein segment that was emptied of all blood via the side branch catheter. The volume of the segment, measured by injection of saline from a volumetric syringe until the vessel was fully distended, was 0.8 to 1.0 ml.

The thrombus was then produced as follows. Approximately 10-20 µg of $^{125}$I-labeled human fibrinogen, containing 1 to $2 \times 10^6$ cpm, was mixed with fresh blood and aspirated in a 1.0 ml syringe to a volume corresponding to the measured volume of the isolated vein segment. The segment was then emptied by withdrawal of the saline through the side branch catheter and 0.2 ml thrombin (100 NIH U/ml) solution containing 10 mM $CaCl_2$ was injected, followed immediately by the mixture of blood and radioactive fibrinogen. Cotton swabs were then placed over the vessel to absorb blood leaking from the vein segment. In most instances, the clot formed quickly, after which it was allowed to age for 30 minutes. Then both vessel clamps were removed and the leg wound was temporarily closed. The cotton swabs were removed for radioisotope counting and the amount of radioactivity delivered to the clot was calculated by subtracting the swab losses, the radioactivity remaining in the syringe, and the total blood radioactivity from the original amount of radioactivity in the syringe.

The plasminogen activators were administered intravenously as a 10% bolus injection, followed by continuous infusion of the remaining 90% dose over 2 hours via a brachial vein. The animals were anticoagulated with heparin (300 units/kg as an intravenous bolus and a continuous infusion of 60 units/kg/hr until the end of the experiment). Thrombolysis was quantitated 30 minutes after the end of the infusion, by determination of the residual radioactivity in the femoral vein segment. To this end, the thrombosed segment of the femoral vein was removed after careful suturing of both ends and the remaining radioactive material in the vein segment was measured. The degree of lysis was expressed as the difference between the radioactivity originally calculated to be incorporated in the clot and the residual radioactivity in the vein segment and was expressed in %.

Two ml blood samples were drawn on citrate (final concentration 0.01 M) before, at hourly intervals after the start of the infusion and at the end of the experiment. The plasma samples were used for measurement of radioactivity, and for fibrinogen, $\alpha_2$-antiplasmin and antigen determination.

The infusions produced virtually linear dose-response curves, in the absence of systemic fibrinogen or $\alpha_2$-antiplasmin consumption. Linear regression lines were drawn in the same way as in the test with hamsters. The resulting line slopes were expressed as % lysis per mg/kg of compound infused (thrombolytic potency), or as % lysis per µg/ml steady state plasma level of u-PA-related antigen (specific thrombolytic activity). The data are represented in table IV.

In table IV, all data represent means ± SEM. The scu-PA used in the rabbit model was a natural scu-PA, whereas a home-made scu-PA was used in the hamster model. Some statistical data have been indicated.

It can be derived from table IV that the thrombolytic potency of nearly all plasminogen activators tested was comparable, except for t-PA-ΔFE/scu-PA-e which had a significantly higher thrombolytic potency in all three animal models. Comparison with table III learns that this effect must be ascribed to a delayed clearance in combination with a rather high specific thrombolytic activity. A delayed clearance was also found in two other plasminogen activators tested but they had no outstanding thrombolytic potency as a result of a reduced specific thrombolytic activity.

14

Example V

Thrombolysis with bolus injection of t-PA-ΔFE/scu-PA, Activase and Saruplase in a hamster pulmonary embolism model

A test was made with bolus injection of 0.25 to 1.0 mg/kg Saruplase, 0.008 to 0.064 mg/kg t-PA-ΔFE/scu-PA-e and 0.062 to 1.0 mg/kg Activase in hamsters with pulmonary embolism. The thrombolytic potency of t-PA-ΔFE/scu-PA-e appeared to be markedly superior to that of Saruplase and Activase. Indeed, the linear regression line slope of % lysis versus mg/kg compound was 1240 ± 290 for t-PA-ΔFE/scu-PA-e as compared to 36 ± 7 for Saruplase and 77 ± 15 for Activase.

EP 0 462 651 A1

TABLE I

Comparative biochemical properties of t-PA/u-PA chimeras and variants

| | Specific activity (IU/mg) | | | | $k_2/K_m$ $(\mu M^{-1}s^{-1})$ | Stimulation by CNBr-fibrinogen (-fold) | Binding to 3.2 mg/ml fibrin (%) | |
|---|---|---|---|---|---|---|---|---|
| | Fibrin plate | | S-2444 | | | | | |
| | single chain | two chain | single chain | two chain | two chain | two chain | single chain | two chain |
| u-PA(CHO) | 120,000± 7,000 | 100,000±22,000 | < 1000 | 100,000±20,000 | 0.088 | 4.9 | 12 ± 6 | 5 ± 8 |
| Saruplase | 180,000±15,000 | 180,000±15,000 | < 1000 | 100,000±17,000 | 0.074 | 4 | - | - |
| t-PA/u-PA-s | 75,000 | 71,000 | < 1000 | 43,000 | 0.070 | - | 59 | 33 ± 7 |
| t-PA/u-PA-e | 170,000±32,000 | 150,000±55,000 | < 1000 | 100,000± 6,900 | 0.080 | 2.2 | 45 ± 14 | 58 ± 7 |
| t-PA-$\Delta$FE/u-PA-e | 99,000±13,000 | 190,000±75,000 | < 1000 | 100,000±26,000 | 0.076 | 2.9 | 22 ± 17 | 19 ± 10 |
| t-PA-$\Delta K_1 \nabla K_2$/u-PA-e | 68,000±16,000 | 120,000±15,000 | < 1000 | 77,000±15,000 | 0.072 | 6.2 | 69 ± 7 | 73 ± 4 |
| t-PA-$\Delta FEK_1 \nabla K_2$/u-PA-e | 200,000±13,000 | 240,000±47,000 | < 1000 | 150,000±39,000 | 0.081 | 2.8 | 46 ± 14 | 47 ± 13 |
| t-PA | 250,000± 5,000 | - | - | - | 0.0006 | 140 | 88 ± 5 | 75 ± 7 |
| Activase[R] | 430,000±54,000 | - | - | - | 0.001(***) | 190(***) | 85 ± 5 | - |

TABLE II

Comparative thrombolytic properties of t-PA/u-PA chimeras and variants in human plasma in vitro

In vitro plasma clot lysis

| | single chain form | | two chain form | |
|---|---|---|---|---|
| | $C_{50}$ ($\mu$g/ml) | Residual fibrinogen (%) | $C_{50}$ ($\mu$g/ml) | Residual fibrinogen (%) |
| u-PA (CHO) | 1.70 | 80 | 0.58 | 35 |
| Saruplase | 1.40 | 80 | 0.33 | 30 |
| t-PA/u-PA-s | 0.60 | 90 | 0.50 | 65 |
| t-PA/u-PA-e | 0.70 | 83 | 0.46 | 70 |
| t-PA-$\Delta$FE/u-PA-e | 0.90 | 77 | 0.37 | 58 |
| t-PA-$\Delta K_1 \nabla K_2$/u-PA-e | 0.86 | 82 | 0.52 | 50 |
| t-PA-$\Delta FEK_1 \nabla K_2$/u-PA-e | 0.53 | 84 | 0.46 | 60 |
| t-PA | 0.50 | 100 | 0.50 | - |
| Activase[R] | 0.20 | 100 | - | - |

EP 0 462 651 A1

TABLE III

Summary of comparative pharmacokinetic properties of single chain t-PA/u-PA chimeras and variants in animal models in vivo.

| | Rabbits | | Hamsters | | Baboons | |
|---|---|---|---|---|---|---|
| | $t\frac{1}{2}\alpha$ (min) | Clp (ml/min) | $t\frac{1}{2}\alpha$ (min) | Clp (ml/min) | $t\frac{1}{2}\alpha$ (min) | Clp (ml/min) |
| scu-PA | 2.0 | 20 ± 2 | 2.7 | 1.1 ± 0.2 | - | - |
| Saruplase | 1.7 | 26 ± 3 | 2.0 | 1.8 ± 0.2 | 2.7 ± 0.5 | 340 ± 44 |
| t-PA/scu-PA-s | 1.6 | 54 ± 3 | 1.4 | 1.1 ± 0.2 | - | - |
| t-PA/scu-PA-e | 1.3 | 66 ± 6 | 1.4 | 1.2 ± 0.2 | - | - |
| t-PA-$\Delta$FE/scu-PA-e | 7 | 2.6 ± 0.2* | 8 | 0.3 ± 0.04* | 13 ± 2 | 34 ± 5* |
| t-PA-$\Delta K_1 \nabla K_2$/scu-PA-e | 4.5 | 7.1 ± 1.4 | 8 | 0.3 ± 0.03 | - | - |
| t-PA-$\Delta FEK_1 \nabla K_2$/scu-PA-e | - | - | 8 | 0.7 ± 0.2 | - | - |
| t-PA | - | - | 2.4 | 5.1 ± 0.7 | - | - |
| Activase [R] | 1.1 | 37 ± 6 | 1.5 | 2.8 ± 0.2 | - | - |

*: p < 0.01 versus Saruplase

TABLE IV
Summary of comparative thrombolytic properties of single chain t-PA/u-PA chimeras and variants in animal models in vivo.

| | Rabbits with jugular vein thrombosis Regression line slope | | Hamsters with pulmonary embolism Regression line slope | | Baboons with femoral vein thrombosis Regression line slope | |
|---|---|---|---|---|---|---|
| | (% lysis per mg/kg compound) | (% lysis per µg/ml plasma level) | (% lysis per mg/kg compound) | (% lysis per µg/ml plasma level) | (% lysis per mg/kg compound) | (% lysis per µg/ml plasma level) |
| scu-PA | 10 ± 1 | 12 ± 4 | 8 ± 1 | 8 ± 1 | - | - |
| Saruplase | 16 ± 8 | 79 ± 9 | 30 ± 3 | 88 ± 15 | 41 ± 8 | 250 ± 260 |
| t-PA/scu-PA-s | 10 ± 0.4 | 28 ± 3 | 25 ± 5 | 8 ± 2 | - | - |
| t-PA/scu-PA-e | 8 ± 1 | 26 ± 7 | 52 ± 8 | 27 ± 12 | - | - |
| t-PA-$\Delta$FE/scu-PA-e | 133 ± 26***+ | 22 ± 3*++ | 510 ± 100*+ | 140 ± 36 | 140 ± 39 | 34 ± 1 |
| t-PA-$\Delta k_1 \nabla k_2$/scu-PA-e | 11 ± 3 | 8 ± 9 | 23 ± 4 | 6 ± 2 | - | - |
| t-PA-$\Delta$FE$k_1 \nabla k_2$/scu-PA-e | - | - | 42 ± 7 | 26 ± 1 | - | - |
| t-PA | - | - | 30 ± 6 | 88 ± 22 | - | - |
| Activase | 41 ± 9 | 190 ± 46 | 82 ± 24 | 330 ± 81 | - | - |

* $p < 0.01$ vs Saruplase; ** $p < 0.05$ versus Saruplase
+ $p < 0.01$ vs Activase[R]; ++ $p < 0.05$ versus Activase[R]

## Claims

1. A chimeric plasminogen activator, comprising in its molecule:
   (a) a polypeptide structure derived from urokinase-type plasminogen activator and harboring substantially all enzymatic activity of urokinase-type plasminogen activator, and
(b) a polypeptide structure capable of endowing the chimeric plasminogen activator with improved in vivo trombolytic potency when compared with urokinase-type plasminogen activator.

2. A chimeric plasminogen activator as claimed in claim 1, wherein said polypeptide structure harboring enzymatic activity of urokinase-type plasminogen activator comprises a carboxyterminal serine protease region of single-chain urokinase-type plasminogen activator.

3. The chimeric plasminogen activator as claimed in claim 1, wherein said polypeptide structure harboring enzymatic activity of urokinase-type plasminogen activator comprises amino acid residues 138 through 411 of single-chain urokinase-type plasminogen activator.

4. A chimeric plasminogen activator as claimed in claim 1, wherein said polypeptide structure capable of endowing the chimeric plasminogen activator with improved in vivo trombolytic potency comprises a polypeptide structure capable of reducing clearance of the chimeric plasminogen activator from an organism while relatively maintaining its specific trombolytic activity in vivo.

5. A chimeric plasminogen activator as claimed in claim 1, wherein said polypeptide structure capable of endowing the chimeric plasminogen activator with improved in vivo trombolytic potency has been derived from tissue-type plasminogen activator.

6. A chimeric plasminogen activator as claimed in claim 1, wherein said polymeric structure capable of endowing the chimeric plasminogen activator with improved in vivo trombolytic potency embraces amino acid residues 1 through 3 and 87 through 274 of a tissue-type plasminogen activator molecule.

7. A chimeric plasminogen activator as claimed in claim 1, comprising in its molecule:
(a) a polypeptide structure embracing amino acid residues 138 through 411 of single-chain urokinase-type plasminogen activator, and
(b) a polypeptide structure embracing amino acid residues 1 through 3 and 87 through 274 of tissue-type plasminogen activator.

8. A DNA segment encoding the chimeric plasminogen activator as claimed in claim 1.

9. An expression vector containing a DNA segment as claimed in claim 8.

10. Prokaryotic or eukaryotic cells, transformed or transfected with expression vectors as claimed in claim 9 and capable of expressing the chimeric plasminogen activator claimed in claim 1.

11. A method of producing a chimeric plasminogen activator comprising in its molecule:
(a) a polypeptide structure derived from urokinase-type plasminogen activator and harboring substantially all enzymatic activity of that urokinase-type plasminogen activator, and
(b) a polypeptide structure capable of endowing the chimeric plasminogen activator with improved in vivo trombolytic potency compared with urokinase-type plasminogen activator, said method comprising the steps of:
- constructing a DNA segment encoding said chimeric plasminogen activator,
- transfecting prokaryotic or eukaryotic cells with said expression vector,
- culturing said transfected cells, and
- harvesting a culture medium containing said chimeric plasminogen activator.

12. A pharmaceutical composition for use in the treatment of thromboembolic diseases, said composition comprising an effective amount of a chimeric plasminogen activator in combination with a pharmaceutically acceptable excipient, said chimeric plasminogen activator comprising in its molecule:
(a) a polypeptide structure derived from urokinase-type plasminogen activator and harboring substantially all enzymatic activity of urokinase-type plasminogen activator, and
(b) a polypeptide structure capable of endowing the chimeric plasminogen activator with improved in vivo thrombolytic potency compared with urokinase-type plasminogen activator.

13. A pharmaceutical composition as claimed in claim 12, wherein said polypeptide structure harboring enzymatic activity of urokinase-type plasminogen activator substantially comprises a carboxyterminal region of single-chain urokinase-type plasminogen activator.

14. A pharmaceutical composition as claimed in claim 12, wherein said polypeptide structure harboring enzymatic activity of urokinase-type plasminogen activator embraces amino acid residues 138 through 411 of single-chain urokinase-type plasminogen activator.

15. A pharmaceutical composition as claimed in claim 12, wherein said polypeptide structure capable of endowing the chimeric plasminogen activator with improved in vivo thrombolytic potency comprises a polypeptide structure capable of reducing clearance of chimeric plasminogen activator from an organism while relatively maintaining its specific thrombolytic activity in vivo.

16. A pharmaceutical composition as claimed in claim 12, wherein said polypeptide structure capable of endowing the chimeric plasminogen activator with improved in vivo thrombolytic potency has been derived from tissue-type plasminogen activator.

17. A pharmaceutical composition as claimed in claim 12, wherein said polypeptide structure capable of endowing the chimeric plasminogen activator with improved in vivo thrombolytic potency embraces amino acid residues 1 through 3 and 87 through 274 of a tissue-type plasminogen activator molecule.

18. A pharmaceutical composition comprising an effective amount of a chimeric activator and a pharmaceutically acceptable excipient, said chimeric plasminogen activator comprising in its molecule:
   (a) a polypeptide structure embracing amino acid residues 138-411 of single-chain urokinase-type plasmingen activator, and
   (b) a polypeptide structure embracing amino acid residues 1 through 3 and 87 through 274 of tissue-type plasminogen activator.

19. A method of treating thromboembolic diseases, comprising administering a chimeric plasminogen activator to a patient in need thereof, said chimeric plasminogen activator comprising in its molecule:
   (a) a polypeptide structure derived from urokinase-type plasminogen activator and harboring substantially all enzymatic activity from urokinase-type plasminogen activator, and
   (b) a polypeptide structure capable of endowing the chimeric plasminogen activator with improved in vivo thrombolytic potency compared with urokinase-type plasminogen activator.

20. A method of treating thromboembolic diseases as claimed in claim 19, wherein said polypeptide structure harboring enzymatic activity of urokinase-type plasminogen activator comprises a carboxyterminal serine protease region of single-chain urokinase-type plasminogen activator.

21. A method of treating thromboembolic diseases as claimed in claim 19, wherein said polypeptide structure harboring enzymatic activity of urokinase-type plasminogen activator comprises amino acid. residues 138 through 411 of single-chain urokinase-type plasminogen activator.

22. A method of treating thromboembolic diseases as claimed in claim 19, wherein said polypeptide structure capable of endowing the chimeric plasminogen activator with improved in vivo thrombolytic potency comprises a polypeptide structure capable of reducing clearance of the chimeric plasminogen activator from an organism while relatively maintaining its specific thrombolytic activity in vivo.

23. A method of treating thromboembolic diseases as claimed in claim 19, wherein said polypeptide structure capable of endowing the chimeric plasminogen activator with improved in vivo thrombolytic potency has been derived from tissue-type plasminogen activator.

24. A method cf treating thromboembolic diseases as claimed in claim 19, wherein said polypeptide structure capable of endowing the chimeric plasminogen activator with improved in vivo thrombolytic potency embraces amino acid residues 1 through 3 and 87 through 274 of a tissue-type plasminogen activator molecule.

25. A method of treating thromboembolic diseases as claimed in claim 19, wherein said chimeric plasminogen activator comprises in its molecule:

21

(a) a polypeptide structure embracing amino acid residues 138 through 411 of single-chain urokinase-plasminogen activator, and
(b) a polypeptide structure embracing amino acid residues 1 through 3 and 87 through 274 of tissue-type plasminogen activator.

FIG.1

FIG.2

## FIG.3

t-PA/u-PA–S

| | F | E | $K_1$ | $K_2$ | . u-PA | |

$S^1$          $T^{263} L^{144}$          $L^{411}$

t-PA/u-PA–e

| | F | E | $K_1$ | $K_2$ | . u-PA | |

$S^1$          $F^{274} S^{138}$          $L^{411}$

t-PAΔ$K_1$∇$K_2$/u-PA – e

| | F | E | $K_2$ | $K_2$ | u-PA | |

$S^1$     $T^{91} C^{180}$   $C^{261} S^{174}$

t-PAΔFE/u-PA – e

| |   | | $K_1$ | $K_2$ | u-PA | |

$S^1$ $Q^3$          $D^{87}$

t-PAΔFE$K_1$∇$K_2$/u-PA–e

| |   | | $K_2$ | $K_2$ | u-PA | |

$S^1$ $Q^3$          $D^{87} T^{91} C^{180}$

European Patent
Office

PARTIAL EUROPEAN SEARCH REPORT
which under Rule 45 of the European Patent Convention
shall be considered, for the purposes of subsequent
proceedings, as the European search report

Application Number

EP 91 20 1447

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 277 313 (CIBA-GEIGY AG) * Claims, in particular 5,6,8,9; page 2, lines 43-47 * | 1,2,4,5 ,8-13 | C 12 N 15/58<br>C 12 N 9/72<br>C 12 N 5/10<br>A 61 K 37/54 |
| Y | WO-A-8 900 197 (GENENTECH, INC.) * Abstract; page 5, line 22 - page 7, line 32; page 50, first paragraph; claims * | 1-18 | |
| Y | GENE, vol. 68, no. 2+index, 1988, pages 205-212, Elsevier Science Publishers B.V.(Biomedical Division), Amsterdam, NL; N.K. KALYAN et al.: "Construction and expression of a hybrid plasminogen activator gene with sequences from non-protease region of tissue-type plasminogen activator (t-PA) and protease region of urokinase (u-PA)" * Abstract; page 211, left-hand column, paragraph 2 *            -/- | 1-18 | |

**TECHNICAL FIELDS SEARCHED (Int. Cl.5)**

C 12 N

## INCOMPLETE SEARCH

The Search Division considers that the present European patent application does not comply with the provisions of the European Patent Convention to such an extent that it is not possible to carry out a meaningful search into the state of the art of some of the claims

Claims searched completely : 1-18
Claims searched incompletely :
Claims not searched : 19-25
Reason for the limitation of the search:

Method for treatment of the human
or animal body by surgery or therapy
(See art. 52(4) of the European
Patent Convention)
A therapeutic method comprising administration
of a chimeric plasminogen activator to patients
suffering thromboembolic diseases.

| Place of search | Date of completion of the search | Examiner |
|---|---|---|
| THE HAGUE | 12-08-1991 | VAN DER SCHAAL C.A.M. |

European Patent
Office

PARTIAL EUROPEAN SEARCH REPORT

Application Number

EP  91 20 1447

| | DOCUMENTS CONSIDERED TO BE RELEVANT | | CLASSIFICATION OF THE APPLICATION (Int. Cl.5) |
|---|---|---|---|
| Category | Citation of document with indication, where appropriate, of relevant passages | Relevant to claim | |
| Y | EMBO JOURNAL, vol. 7, no. 9, 25th August 1988, pages 2731-2739, IRL Press Ltd, Oxford, GB; M.-J. GETHING et al.: "Variants of human tissue-type plasminogen activator that lack specific structural domains of the heavy chain" * Abstract; figure 1; page 2735, right-hand column paragraph 3; page 2736, "Discussion" * --- | 1-18 | |
| Y | THE JOURNAL OF BIOLOGICAL CHEMISTRY, vol. 263, no. 4, 5th February 1988, pages 1599-1602, The American Society for Biochemistry and Molecular Biology, Inc., Baltimore, US; M.J. BROWNE et al.: "A tissue-type plasminogen activator mutant with prolonged clearance in vivo" * Abstract; page 1601, "Discussion" first sentence * --- | 1-18 | TECHNICAL FIELDS SEARCHED (Int. Cl.5) |
| D,Y | THE JOURNAL OF BIOLOGICAL CHEMISTRY, vol. 263, no. 35, 15th December 1988, pages 19083-19091, The American Society for Biochemistry and Molecular Biology, Inc., Baltimore, US; H.R. LIJNEN et al.: "Characterization of a chimeric plasminogen activator consisting of amino acids 1 to 274 of tissue-type plasminogen activator and amino acids 138 to 411 of single-chain urokinase-type plasminogen activator" * Abstract * --- | 1-18 | |
| P,X | THROMBOSIS AND HAEMOSTASIS, vol. 64, no. 1, 13th August 1990, pages 53-60, Stuttgart, DE; L. NELLES et al.: "Characterization of domain deletion and/or duplication mutants of a recombinant chimera of tissue-type plasminogen activator and urokinase-type plasminogen activator (rt-PA/u-PA)" * The whole a rticle * ----- | 1-18 | |